# EUROPEAN PATENT APPLICATION

(11) **EP 2 221 057 A1**
(43) Date of publication of application: **25.08.2010**
(21) Application number: 08851416.1
(22) Date of filing: 19.11.2008
(51) Int. Cl.: A61K 31/688, A23L 1/30, A61K 8/55, A61P 25/02, A61Q 19/00

(54) **SENSE-IMPROVING AGENT**

(30) Priority: 19.11.2007 JP 2007299877; 19.11.2007 JP 2007299878
(71) Applicant: Snow Brand Milk Products Co., Ltd., Sapporo-shi Hokkaido 065-0043 (JP)
(72) Inventor: WATANABE, Tatsuya, Kawagoe-shi Saitama 350-1165 (JP); HARUTA, Yuko, Kawagoe-shi Saitama 350-1165 (JP); KATO, Ken, Kawagoe-shi Saitama 350-1165 (JP); YOSHIOKA, Toshimitsu, Kawagoe-shi Saitama 350-1165 (JP)
(74) Representative: Jones, Helen M.M.
(86) International application number: PCT/JP2008/071005
(87) International publication number: WO 2009/066685

(57) **Abstract**

Disclosed are: sense-improving agent which comprises, as an active ingredient, a phospholipid or a sphingosine-containing phospholipid and/or a derivative thereof, particularly a sphingomyelin, and which has an effect of improving the dulling of senses at a periphery when ingested orally or directly applied to the skin; and a sense-improving food, beverage, feed or cosmetic comprising the sense-improving agent. The phospholipid to be used may be a chemically synthesized phospholipid or a naturally occurring phospholipid, preferably a phospholipid derived from an edible material such as soybean and egg yolk, particularly preferably a phospholipid derived from milk.

## Description

### TECHNICAL FIELD

The present invention relates to a skin sensitivity improving agent that includes a phospholipid or a sphingosine-containing phospholipid and/or a derivative thereof, particularly sphingomyelin as an active ingredient and has an effect improving deterioration in peripheral sensation, and further relates to a sensation-improving food, drink, feed, or cosmetic that includes the skin sensitivity improving agent.

### BACKGROUND ART

In recent years, an increase in age-related diseases such as osteoporosis, dementia and the like has become a serious social issue. Various drugs have been developed to prevent or improve such age-related diseases. However, since drugs always need to take side effects into consideration, in recent years, attempts have been made to prevent or improve age-related diseases through a change in eating habits or intake of a specific food ingredient. For example, it is known that osteoporosis may be prevented or improved by intake of a basic protein contained in cow milk (see Patent Document 1). Furthermore, a dementia therapeutic agent that prevents or improves Alzheimer-type dementia and comprises sphingomyelin which is a phospholipid containing relatively abundantly in cow milk as an active ingredient has been known (see Patent Document 2).

Deterioration in peripheral sensation can be given as one of the age-related symptoms. The deterioration in peripheral sensation occurs due to not only aging, but also, diseases such as diabetes and the like. Deterioration in peripheral sensation may occur following troubles; for example, as it can't feel hot rightly when touching a hot object, a risk of suffering bums or the like increases or the discovery of an injury becomes delay due to deterioration in pain sensation. In recent year, in order to prevent such a risk, studies that improve deterioration in peripheral sensation due to ageing or diseases have been conducted. For example, it has been reported that sphingomyelinase or phosphatidylcholine-specific phospholipase C, which are an enzyme that increases biosynthesis of exogenous or endogenous ceramide promotes differentiation of P-12 cells which is nerve model cells through established fibroblast cell strain 3T3 cells (see Non-patent Document 1). As for promoting differentiation of nerve model cells, it suggests to exhibit an effect to the improvement of deterioration in peripheral sensation. However, since ceramide and the above enzyme are not a food ingredient, it is necessary to take account of safety issues.
The above experimental results were obtained when directly applying the enzyme to skin, and thus an effect achieved via oral intake is unknown. In the circumstances, a safe agent that is effective to improve deterioration in peripheral sensation through daily intake or skin application has been desired.
Though sphingomyelin is contained large amounts of 20 to 30 wt% of phospholipids contained in milk, the functions of sphingomyelin have been studied only at a cellular level, and the physiological functions of sphingomyelin in a living body have been known to only a small extent. Therefore, the efficiency of sphingomyelin as one ingredient of a nutrient has not been identified until now. Sphingomyelin has been known to use for an antiphlogistic/analgesic external preparation, a lipid digestion absorption improver, a therapeutic agent for intestinal motor dysfunction-related disease, and the like (see Patent Documents 3 to 5). However, whether or not sphingomyelin has an effect of preventing or improving deterioration in peripheral sensation has not been known.
Patent Document 1: JP-A-H08-151331
Patent Document 2: JP-A-2003-146883
Patent Document 3: JP-A-H05-186330
Patent Document 4: JP-A-H11-269074
Patent Document 5: JP-A-2003-252765
Non-patent Document 1: Norimichi Nakahata and Satoko Okubo, Mechanism of Development of Physiological Effect of Ceramide Having Skin Protective Function, Annual Report of Cosmetology, vol. 10, 2002

### DISCLOSURE OF THE INVENTION

### PROBLEMS TO BE SOLVED BY THE INVENTION

An object of the present invention is to provide a safe and skin sensitivity improving agent that is effective to improve deterioration in peripheral sensation through daily intake or skin application. Another object of the present invention is to provide a sensation-improving food, drink, feed, or cosmetic that is effective to improve deterioration in peripheral sensation through oral intake or skin application.

### MEANS FOR SOLVING THE PROBLEMS

The inventors of the present invention, in consideration of those problems, searched for a safe component that exhibits an excellent improvement effect to the sensation deterioration. As a result, the inventors found that deterioration in sensation, particularly peripheral sensation, can be improved by oral intake or direct application of a phospholipid or a sphingosine-containing phospholipid and/or a derivative thereof, particularly sphingomyelin, onto the skin. The inventors thus completed a skin sensitivity improving agent by utilizing a phospholipid or a sphingosine-containing phospholipid and/or a derivative thereof (particularly sphingomyelin) as an active ingredient. The inventors also found that a sensation-improving food, drink, feed, or cosmetic can be obtained by adding the skin sensitivity improving agent to food, drink, or feed, respectively. These findings have led to completion of the present invention.

### EFFECTS OF THE INVENTION

The present invention thus provides a skin sensitivity improving agent that includes a phospholipid or a sphingosine-containing phospholipid and/or a derivative thereof, particularly sphingomyelin, as an active ingredient, and a sensation-improving food, drink, feed, or cosmetic that includes a phospholipid. The skin sensitivity improving agent according to the present invention exhibits an effect that improves deterioration in peripheral sensation.

### BEST MODE FOR CARRYING OUT THE INVENTION

The present invention is **characterized in that** a phospholipid or a sphingosine-containing phospholipid and/or a derivative thereof (particularly sphingomyelin) is used as an active ingredient. A chemically synthesized phospholipid or a naturally-derived phospholipid may be used as the phospholipid. It is preferable to use a phospholipid derived from food, such as soybean, egg yolk or the like. It is particularly preferable to use a milk-derived phospholipid. As the milk-derived phospholipid, a phospholipid prepared from mammalian milk, such as cow milk, goat milk, ewe milk, human milk or the like may be used. The phospholipid may also be prepared from a milk material, such as butter serum, buttermilk or the like prepared from those mammalian milks. The phospholipid needs not necessarily be purified. A milk-derived phospholipid-containing composition that contains a phospholipid in an amount of 30 wt% or more in the total solid may also be used. For example, in the present invention the milk-derived phospholipid-containing composition may be obtained by treating milk or a milk material using a microfiltration (MF) membrane having a pore diameter of 0.1 to 2.0 µm or an ultrafiltration (UF) membrane having a molecular weight cut-off of 5 to 500 kDa. The milk-derived phospholipid-containing composition of the invention may also be obtained by adding an acid to milk or a milk material to adjust the pH thereof to 4.0 to 5.0, removing a casein protein as a precipitate, and treating the resultant using an MF membrane having a pore diameter of 0.1 to 2.0 µm or a UF membrane having a molecular weight cut-off of 5 to 500 kDa. Inexpensive milk-derived phospholipid-containing composition that is prepared from milk and has a high phospholipid content of 30 wt% or more is commercially available, and may also be used in the present invention. Soybean lecithin and egg-yolk lecithin are also commercially available as other phospholipids.

The sphingosine-containing phospholipid and/or a derivative thereof, particularly sphingomyelin, may be used in purified form, or may be used as a sphingomyelin-containing composition. The sphingomyelin is contained in an animal brain or milk fat to a large extent. It is preferable to use milk-derived sphingomyelin for putting the present invention into practice. Milk-derived sphingomyelin may be prepared using raw milk, a whey protein concentrate (WPC) or the like as the raw material. A method of preparing the sphingomyelin-containing composition from raw milk, a WPC, or the like may include known methods, such as a method of extracting the composition with ether or acetone, a method of using a water-soluble fraction that contains butter serum or butter curds obtained by melting butter with heat, or the like. The resulting sphingomyelin-containing composition may be purified by dialysis, ammonium sulfate fractionation, gel filtration, isoelectric precipitation, ion-exchange chromatography, solvent fractionation, ultrafiltration (UF), microfiltration (MF), or the like to increase the purity of sphingomyelin. Sphingomyelin and the sphingomyelin-containing composition may be appropriately used in the form of liquid, powder, tablets, or the like, and directly administered orally.

The milk-derived phospholipid, the milk-derived phospholipid-containing composition, soybean lecithin, egg-yolk lecithin, or the like may be directly used as the skin sensitivity improving agent according to the present invention. The skin sensitivity improving agent may further mixed a raw material that is normally used for drugs, food, drink and feed, such as saccharides, lipids, proteins, vitamins, minerals, flavors or the like, in addition to phospholipid. The skin sensitivity improving agent may be prepared into a powdered drug, granules, a tablet, a capsule, a drinkable preparation or the like by a conventional method. The skin sensitivity improving agent may also be used in common application form such as emulsion, cream, lotion, massage mask or the like. The skin sensitivity improving agent in application form may be prepared by a conventional method while appropriately adding the phospholipid or the phospholipid-containing composition, such as milk-derived phospholipid-containing composition, used as an active ingredient in the present invention, and may be used as a cosmetic. Another component, e.g., ceramide, sphingomyelinase or the like, that exhibits a sensation-improving effect may be used in combination with the phospholipid.

In the mouse experiments described later, the peripheral sensation was improved by orally administering the phospholipid-containing composition in an amount of 100 mg/kg or more, and preferably 250 mg/kg or more. Therefore, deterioration in sensation, particularly peripheral sensation, is expected to be improved when an adult generally takes the phospholipid-containing composition in an amount of 100 mg/day or more, and preferably 250 mg/day or more, and thus it is desired to take the above necessary quantity. Since the milk-derived phospholipid-containing composition contains the milk-derived phospholipid in an amount of 30% or more, a sensation-improving effect is expected to be achieved by taking the milk-derived phospholipid in an amount of about 30 mg or more. When applying the phospholipid-containing composition to skin, the milk-derived phospholipid is contained 0.01 to 50 wt% in the phospholipid-containing composition (skin liniment), and preferably 0.1 to 20 wt%.

From the results for the human experiments described later, deterioration in sensation, particularly peripheral sensation, is expected to be improved when an adult takes the sphingosine-containing phospholipid and/or a derivative thereof, particularly sphingomyelin, in an amount of 4 mg/day or more, and preferably 8 mg/day or more. Therefore, the dosage of sphingomyelin or the sphingomyelin-containing composition may be determined taking the above results into consideration. When applying sphingomyelin or the sphingomyelin-containing composition to skin, the content of the sphingosine-containing phospholipid and/or a derivative thereof, particularly sphingomyelin, in the skin liniment may (but not specifically limited to) be 0.001 to 30 wt%, and preferably 0.1 to 10 wt%.

The sensation-improving food or drink according to the present invention may be prepared by adding the skin sensitivity improving agent that includes the phospholipid or the sphingosine-containing phospholipid and/or a derivative thereof, particularly sphingomyelin, as an active ingredient to normal food or drink, such as yogurt, milk-based drink, wafer, dessert or the like.
It is preferable that the sensation-improving food or drink contain the phospholipid-containing composition in an amount of 1 to 3000 mg per 100 g of the food or drink though depending on the type of food or drink so that an adult takes the phospholipid-containing composition in an amount of 100 mg/day or more.
It is preferable that the sensation-improving food or drink contain the sphingosine-containing phospholipid and/or a derivative thereof, particularly sphingomyelin, in an amount of 0.5 to 2000 mg per 100 g of the food or drink though depending on the type of food or drink so that the sphingosine-containing phospholipid and/or a derivative thereof, particularly sphingomyelin is taken in an amount of 4 mg or more. The sensation-improving feed according to the present invention may be prepared by adding the phospholipid or the sphingosine-containing phospholipid and/or a derivative thereof, particularly sphingomyelin, to normal feed, such as livestock food, pet food or the like. When preparing the sensation-improving feed adding the milk-derived phospholipid-containing composition, it is preferable that the sensation-improving feed contain the milk-derived phospholipid-containing composition in an amount of 1 to 5000 mg per 100 g of the feed so that the phospholipid is taken in an amount of 30 mg/day or more, for example. It is preferable that the sensation-improving feed contain the sphingosine-containing phospholipid and/or a derivative thereof, particularly sphingomyelin, in an amount of 0.5 to 2000 mg per 100 g of the feed so that the sphingosine-containing phospholipid and/or a derivative thereof, particularly sphingomyelin, is taken in an amount of 2 mg or more.

The method of mixing the phospholipid-containing composition or the sphingosine-containing phospholipid and/or a derivative thereof, particularly sphingomyelin may not be particularly limited. For example, the composition is suspended or dissolved in deionized water, and the mixture is stirred and prepared in the form of a drug, food, drink, or feed. The stirring/mixing conditions are not particularly limited insofar as the composition is uniformly mixed. An ultra-disperser, a TK-homomixer, or the like may be used for the stirring/mixing. The solution containing the composition may optionally be concentrated using a reverse osmosis (RO) membrane or freeze-dried so that the solution can be easily used for a drug, food, drink, or feed. A sterilization treatment conventionally used in the production of drugs, food, drink, or feed may be employed in the present invention. A powdery product may be subjected to dry-heat sterilization. Therefore, drugs, food, drink, and feed in various forms (e.g., liquid, gel, powder, granules or the like) that contain the phospholipid-containing composition or the sphingosine-containing phospholipid and/or a derivative thereof, particularly sphingomyelin, according to the present invention can be produced.

The present invention is further described below by way of examples and test examples. Note that the following examples should not be construed as limiting the present invention.

### Example 1

### (Preparation 1 of phospholipid-containing composition)

A 25% solution of a butter serum powder ("SM2" manufactured by Corman) was provided. 5N hydrochloric acid was added to the solution to adjust the pH of the solution to 4.5. The solution was allowed to stand at 50°C for one hour to precipitate a casein protein. After removing the precipitate using a filter press, the resulting aqueous solution was filtered through an MF membrane with a pore diameter of 1.0 µm (manufactured by SCT) to obtain a concentrate fraction. The concentrate fraction was freeze-dried to obtain a phospholipid-containing composition. The milk-derived phospholipid-containing composition contained 53% of lipid, 31% of phospholipid, 24% of protein, 15% of carbohydrate, and 8% of ash based on the total solid content. The milk-derived phospholipid-containing composition may be directly used as the skin sensitivity improving agent according to the present invention.

### Example 2

### (Preparation 2 of phospholipid-containing composition)

A 20% solution of a buttermilk powder (manufactured by Snow Brand Milk Products Co., Ltd.) was provided. 2N hydrochloric acid was added to the solution to adjust to pH 4.5. The solution was allowed to stand at 45°C for 30 minutes to precipitate a casein protein. After removing the precipitate using a clarifier, the obtained supernatant was filtered through an MF membrane with a pore diameter of 0.1 µm (manufactured by SCT) to obtain a concentrate fraction. The concentrate fraction was freeze-dried to obtain a milk-derived phospholipid-containing composition. The milk-derived phospholipid-containing composition contained 62% of lipid content of, 38% of phospholipid, 15% of protein content of, 18% of carbohydrate, and 5% of ash based on the total solid content. The milk-derived phospholipid-containing composition may be directly used as the skin sensitivity improving agent according to the present invention.

### Example 3

### (Preparation 1 of sphingomyelin)

A reaction solution obtained by reacting a protease with a 10% aqueous solution of a whey protein concentrate (WPC) was extracted with a chloroform-methanol (2: 1) solution, then concentrated, and extracted with acetone to obtain a phospholipid fraction.
The obtained phospholipid fraction was purified by silica gel column chromatography, subjected to stepwise extraction with a chloroform-methanol solution, and freeze-dried to obtain purified sphingomyelin. The purified product was fractionated by thin-layer chromatography, colored using a Dittmer reagent, and quantified using a densitometer. The sphingomyelin content was found to be 95.2%. The resulting sphingomyelin may be directly used as the skin sensitivity improving agent.

### Example 4

### (Preparation 2 of sphingomyelin)

A sphingomyelin material ("Phospholipid 700" manufactured by Fonterra) with a sphingomyelin content of 25% was extracted with acetone to obtain a phospholipid fraction. The obtained phospholipid fraction was purified by silica gel column chromatography, subjected to stepwise extraction with a hexane-ethanol solution, and freeze-dried to obtain purified sphingomyelin. The purified product was fractionated by thin-layer chromatography, colored using a Dittmer reagent, and quantified using a densitometer. The sphingomyelin content was found to be 78.2%. The resulting sphingomyelin may be directly used as the skin sensitivity improving agent.

### Test Example 1

### (Confirmation of cell differentiation-promoting activity - milk-derived phospholipid-containing composition)

3T3 cells, which is fibroblast cell strain known to be present in skin, were cultured for two days under the condition that the milk-derived phospholipid-containing composition of Example 1 or 2 was added at a concentration of 200 µg/l (Example 1: Group A, Example 2: Group B). As a control, 3T3 cells were cultured for two days without adding the milk-derived phospholipid-containing composition obtained in Example 1 or 2 (Group C). PC-12 cells (nerve model cells) were cultured using the culture supernatant thereof, and morphological differentiation of the PC-12 cells was observed. As the results, the PC-12 cells were highly differentiated when using either culture supernatant of the group A or B. The above experiment was repeated several times, and the differentiation rate was observed through an optical microscope. It was confirmed that 95% or more of the cells were differentiated when using the culture supernatant of the group A or B. On the other hand, the PC-12 cells were not differentiated when using the culture supernatant of the group C. No differentiation was observed through an optical microscope even though the experiment was repeated several times. It was thus confirmed that the milk-derived phospholipid-containing compositions obtained in Examples 1 and 2 promote differentiation of the PC-12 cells which was nerve model cells.

### Test Example 2

### (Confirmation of cell differentiation-promoting activity - sphingomyelin)

3T3 cells, which is fibroblast cell strain known to be present in skin, were cultured for two days under the condition that the sphingomyelin obtained in Example 3 was added at a concentration of 200 µg/l (group A). As a control, 3T3 cells were cultured for two days without adding the sphingomyelin (group B). PC-12 cells, which were nerve model cells, were cultured using the culture supernatant thereof, and morphological differentiation of the PC-12 cells was observed. The PC-12 cells were highly differentiated when using the culture supernatant of the group A. The above experiment was repeated several times, and the differentiation rate was observed through an optical microscope. It was confirmed that 94% or more of the cells were differentiated when using the culture supernatant of the group A. On the other hand, the PC-12 cells were not differentiated when using the culture supernatant of the group B. No differentiation was observed using an optical microscope even though the experiment was repeated several times. It was thus confirmed that the sphingomyelin obtained in Example 3 promotes differentiation of the PC-12 cells which was nerve model cells.

### Test Example 3

### (Confirmation of sensation-improving effect by animal experiments)

A sensation-improving effect by thermal stimulation was evaluated by the hot plate test that is a thermal stimulation behavioristic approach developed by Woolfe and MacDonald. Three groups of 24-week-old hairless mice (HR-1) were used (one group: ten mice). The milk-derived phospholipid-containing composition obtained in Example 1 was orally administered to each mouse using a sonde in an amount of 0, 100, or 250 mg/kg mouse weight once daily for four weeks. The mouse was placed on a hot plate at 54°C upon completion of the dietary administration, and the time elapsed until the mouse made an escape behavior such as taking off the foot from the hot plate, standing up, or jumping was measured. The cut-off time loading maximum intensity in the thermal stimulation was set to 30 seconds. The results are shown in Table 1.

**TABLE 1**

| Milk-derived phospholipid-containing composition of Example 1 | Escape behavior positive reaction time |
|---|---|
| 0 mg | 28.2 ± 0.14 sec |
| 100 mg | 23.8 ± 0.19 sec |
| 250 mg | 20.1 ± 0.32 sec |

As shown in Table 1, the escape behavior reaction time tended to be reduced when administering the milk-derived phospholipid-containing composition obtained in Example 1 in an amount of 100 mg, and was reduced when administering in an amount of 250 mg. It was thus confirmed that deterioration in sensation, particularly peripheral sensation, can be prevented or improved by intake of the milk-derived phospholipid-containing composition.

### Test Example 4

### (Confirmation of sensation-improving effect via oral intake - milk-derived phospholipid-containing composition)

Healthy elderly persons (average age: 75±3) who suffered deterioration in sensation in the hands were divided into following five groups (one group: 10 subjects).
Group A: the subjects did not take a phospholipid-containing composition,
Group B: the subjects took the milk-derived phospholipid-containing composition obtained in Example 1 in an amount of 100 mg,
Group C: the subjects of the took the milk-derived phospholipid-containing composition obtained in Example 1 in an amount of 250 mg,
Group D: the subjects took a commercially available milk-derived phospholipid concentrate ("PL-75" manufactured by Lacprodan) in an amount of 40 mg,
Group E: the subjects took a commercially available milk-derived phospholipid concentrate ("PL-75" manufactured by Lacprodan) in an amount of 100 mg,
Group F: the subjects took a commercially available soybean-derived phospholipid ("LP-20P" manufactured by The Nisshin OilliO Group, Ltd.) in an amount of 40 mg, and
Group G: the subjects took a commercially available soybean-derived phospholipid ("LP-20P" manufactured by The Nisshin OilliO Group, Ltd.) in an amount of 100 mg. The test was conducted for six weeks. The pain sensation in the palm and the arch of the foot was measured with 4 criteria (normal, deteriorations I, II, and III) before the intake and after the intake for 6 weeks with reference to the pain sensation in the medial side of the arm using a pain/touch-pressure sensation measuring device ("Algesiometer" manufactured by Intercross Ltd.) in accordance with the instruction manual. After the intake for 6 weeks, a questionnaire survey on improvement in sensation in the hands was carried out to each elderly person. The results are shown in Tables 2 and 3.

### (Measuring method)

The pain sensation was evaluated using five pins that differ in thickness in combination with five fulcrum positions. The thinnest pin 1 was rolled along the medial side of the arm, and the subject was asked about the degree of normal pain sensation. The pin 1 was then rolled along the palm and the bottom of the foot while sequentially changing the holder fulcrum position to determine the fulcrum position at which the same pain sensation degree as the first pain sensation occurred.

### (Evaluation method)

The Algesiometer is designed so that the pain sensations with same degree occur between when rolling the pin 1 (fulcrum: 50 g) along the medial side of the arm and when rolling the pin 2 (fulcrum: 50 g) along the palm. The pain sensation was evaluated as follows in accordance with the instruction manual. The pain sensation was evaluated by points, and the average points were calculated. Normal (0 points): The same pain sensation occurred when rolling the pin 2 (fulcrum: 50 g). Deterioration I (1 point): The same pain sensation occurred when rolling the pin 1 (fulcrum: 50 g). Deterioration II (2 points): The same pain sensation occurred when rolling the pin 1 (fulcrum: 60 g): Deterioration III (3 points): The same pain sensation occurred when rolling the pin 1 (fulcrum: 70 g):

**TABLE 2-1**

| Hand sensation measurement (before intake) | | | | | |
|---|---|---|---|---|---|
| | Normal | Deterioration I | Deterioration II | Deterioration III | Average value |
| Group A | 0 | 2 | 3 | 5 | 2.3 |
| Group B | 0 | 1 | 5 | 4 | 2.3 |
| Group C | 0 | 1 | 5 | 4 | 2.3 |
| Group D | 0 | 1 | 4 | 5 | 2.4 |
| Group E | 0 | 1 | 5 | 4 | 2.3 |
| Group F | 0 | 2 | 3 | 5 | 2.3 |
| Group G | 0 | 1 | 4 | 5 | 2.4 |

| Hand sensation measurement (after intake for 6 weeks) | | | | | |
|---|---|---|---|---|---|
| | Normal | Deterioration I | Deterioration II | Deterioration III | Average value |
| Group A | 0 | 2 | 4 | 4 | 2.2 |
| Group B | 0 | 3 | 5 | 2 | 1.9 |
| Group C | 2 | 4 | 3 | 1 | 1.3 |
| Group D | 1 | 2 | 4 | 3 | 1.9 |
| Group E | 2 | 2 | 5 | 1 | 1.5 |
| Group F | 0 | 3 | 3 | 1 | 2.1 |
| Group G | 0 | 5 | 4 | 1 | 1.6 |

**TABLE 2-2**

| Foot bottom sensation measurement (before intake) | | | | | |
|---|---|---|---|---|---|
| | Normal | Deterioration I | Deterioration II | Deterioration III | Average value |
| Group A | 0 | 2 | 3 | 5 | 2.3 |
| Group B | 0 | 1 | 4 | 5 | 2.4 |
| Group C | 0 | 1 | 3 | 6 | 2.5 |
| Group D | 0 | 1 | 6 | 3 | 2.2 |
| Group E | 0 | 1 | 4 | 5 | 2.4 |
| Group F | 0 | 2 | 3 | 5 | 2.3 |
| Group G | 0 | 1 | 6 | 3 | 2.2 |

| Foot bottom sensation measurement (after intake for 6 weeks) | | | | | |
|---|---|---|---|---|---|
| | Normal | Deterioration I | Deterioration II | Deterioration III | Average value |
| Group A | 0 | 2 | 3 | 5 | 2.3 |
| Group B | 1 | 4 | 1 | 4 | 1.8 |
| Group C | 2 | 3 | 3 | 2 | 1.5 |
| Group D | 0 | 3 | 6 | 1 | 1.8 |
| Group E | 1 | 3 | 5 | 1 | 1.6 |
| Group F | 0 | 4 | 2 | 4 | 2.0 |
| Group G | 1 | 4 | 2 | 3 | 1.7 |

**TABLE 3**

| Hand sensation | | | |
|---|---|---|---|
| | Worsened | Same | Improved |
| Group A | 3 | 6 | 1 |
| Group B | 1 | 5 | 4 |
| Group C | 0 | 2 | 8 |
| Group D | 0 | 3 | 7 |
| Group E | 0 | 2 | 8 |
| Group F | 1 | 6 | 3 |
| Group G | 0 | 5 | 5 |

| Foot bottom sensation | | | |
|---|---|---|---|
| | Worsened | Same | Improved |
| Group A | 1 | 8 | 1 |
| Group B | 0 | 6 | 4 |
| Group C | 0 | 2 | 8 |
| Group D | 0 | 5 | 5 |
| Group E | 0 | 2 | 8 |
| Group F | 0 | 5 | 5 |
| Group G | 0 | 3 | 7 |

As shown in Tables 2 and 3, the sensation in the palm and the bottom of the foot tended to be improved by intake of the milk-derived phospholipid-containing composition obtained in Example 1 in an amount of 100 mg, and was significantly improved by in an amount of 250 mg. It was also confirmed that the sensation in the palm and the bottom of the foot is improved by intake of the commercially available milk-derived phospholipid concentrate and the commercially available soybean-derived phospholipid concentrate. Deterioration in sensation, particularly peripheral sensation, is expected to be improved when an adult takes the phospholipid-containing composition in an amount of 100 mg/day or more, and preferably 250 mg/day or more.
It was confirmed that the sensation-improving effect can be expected to be achieved with a smaller amount of phospholipid-containing composition when using the phospholipid concentrate with higher purity.

### Test Example 5

### (Sensation-improving effect test through oral intake - sphingomyelin)

Healthy elderly persons (average age: 75±3) who suffered deterioration in sensation in the hands were divided into three groups (one group: 15 subjects). The subjects took the sphingomyelin obtained in Example 4 in an amount of 0 mg, 4 mg, or 8 mg for 12 weeks. The pain sensation in the palm and the arch of the foot was measured with 4 criteria (normal, deteriorations I, II, and III) before the intake and after the intake for 12 weeks with reference to the pain sensation in the medial side of the arm using a pain/touch-pressure sensation measuring device ("Algesiometer" manufactured by Intercross Ltd.) in accordance with the instruction manual. After the intake for 12 weeks, a questionnaire survey on improvement in sensation in the hands was carried out to each elderly person. The measurement method and the evaluation method are the same as those employed in Test Example 4.
The results are shown in Tables 4 and 5.

**TABLE 4**

| Hand sensation measurement (before intake) | | | | | |
|---|---|---|---|---|---|
| Sphingomyelin | Normal | Deterioration I | Deterioration II | Deterioration III | Average value |
| 0 mg | 0 | 2 | 7 | 6 | 2.3 |
| 4 mg | 0 | 3 | 7 | 5 | 2.1 |
| 8 mg | 0 | 2 | 6 | 7 | 2.3 |

| Hand sensation measurement (after intake for 12 weeks) | | | | | |
|---|---|---|---|---|---|
| Sphingomyelin | Normal | Deterioration I | Deterioration II | Deterioration III | Average value |
| 0 mg | 0 | 3 | 8 | 4 | 2.1 |
| 4 mg | 2 | 5 | 6 | 2 | 1.5 |
| 8 mg | 3 | 7 | 3 | 2 | 1.3 |

| Hand Foot bottom sensation measurement (before intake) | | | | | |
|---|---|---|---|---|---|
| Sphingomyelin | Normals | Deterioration I | Deterioration II | Deterioration III | Average value |
| 0 mg | 0 | 2 | 8 | 5 | 2.2 |
| 4 mg | 0 | 3 | 7 | 5 | 2.1 |
| 8 mg | 0 | 2 | 7 | 6 | 2.3 |

| Hand Foot bottom sensation measurement (after intake for 12 weeks) | | | | | |
|---|---|---|---|---|---|
| Sphingomyelin | Normal | Deterioration I | Deterioration II | Deterioration III | Average value |
| 0 mg | 0 | 2 | 7 | 6 | 2.3 |
| 4 mg | 1 | 5 | 7 | 2 | 1.7 |
| 8 mg | 1 | 7 | 4 | 3 | 1.6 |

**TABLE 5**

| Hand sensation | | | |
|---|---|---|---|
| Sphingomyelin | Worsened | Same | Improved |
| 0 mg | 3 | 9 | 3 |
| 4 mg | 2 | 7 | 6 |
| 8 mg | 1 | 4 | 10 |

| Foot sensation | | | |
|---|---|---|---|
| Sphingomyelin | Worsened | Same | Improved |
| 0 mg | 3 | 11 | 1 |
| 4 mg | 1 | 9 | 5 |
| 8 mg | 0 | 7 | 8 |

As shown in Tables 4 and 5, the sensation in the palm and the bottom of the foot tended to be improved by intake of the sphingomyelin in an amount of 4 mg, and was significantly improved by intake of the sphingomyelin in an amount of 8 mg. Deterioration in sensation, particularly in peripheral sensation, is expected to be improved when an adult takes the sphingosine-containing phospholipid and/or a derivative thereof, particularly sphingomyelin, in an amount of 4 mg/day or more, and preferably 8 mg/day or more.

### Example 5

### (Preparation of sensation-improving cosmetic (cream) - milk-derived phospholipid-containing composition)

A sensation-improving cosmetic (cream) was prepared by mixing the skin sensitivity improving agent (milk-derived phospholipid-containing composition) obtained in Example 1 with the raw materials in a ratio shown in Table 6.

**TABLE 6**

| | |
|---|---|
| Glycerol monostearate (self-emulsifiable) | 10.0 |
| Purified lanolin | 6.0 |
| Liquid paraffin | 5.0 |
| Jojoba oil | 5.0 |
| Parabene | 0.3 |
| Milk-derived phospholipid-containing composition (Example 1) | 4.5 |
| Essence | Proper quantity |
| Sterilized ion-exchanged water | Balance (total: 100) |

### Example 6

### (Preparation of sensation-improving cosmetic (cream) - sphingomyelin)

A sensation-improving cosmetic (cream) was produced by mixing the sphingomyelin obtained in Example 3 with the raw materials in a ratio shown in Table 7.

**TABLE 7**

| | |
|---|---|
| Glycerol monostearate (self-emulsifiable) | 10.0 |
| Purified lanolin | 6.0 |
| Liquid paraffin | 5.0 |
| Jojoba oil | 5.0 |
| Parabene | 0.3 |
| Sphingomyelin (Example 3) | 0.3 |
| Essence | Proper quantity |
| Sterilized ion-exchanged water | Balance (total: 100) |

### Test Example 6

### (Sensation-improving effect test through skin application - milk-derived phospholipid-containing composition)

Healthy elderly persons (average age: 75±3) who suffered deterioration in sensation in the hands were divided into groups A and B (one group: 15 subjects). The subjects of the group A were applied a cosmetic (cream) that was produced in the same manner as in Example 5 but did not contain a skin sensitivity improving agent once daily to whole of the hands and the feet thereof, and The subjects of the group B were applied the sensation-improving cosmetic (cream) obtained in Example 5 was applied once daily to whole of the hands and the feet thereof. The application period was six weeks. The pain sensation in the palm and the arch of the foot was measured with 4 criteria (normal, deterioration I, II, and III) before the application and after the application for 6 weeks with reference to the pain sensation in the medial side of the arm using a pain/touch-pressure sensation measuring device ("Algesiometer" manufactured by Intercross Ltd.) in accordance with the instruction manual. After the completion of the application for 6weeks, a questionnaire survey on improvement in sensation in the hands was carried out to each elderly person. The results are shown in Tables 8 and 9. The measurement method was conducted in the same manner as that of Test Example 4.

**TABLE 8**

| Hand sensation measurement (before application) | | | | | |
|---|---|---|---|---|---|
| | Normal | Deterioration I | Deterioration II | Deterioration III | Average value |
| Group A | 0 | 4 | 6 | 5 | 2.1 |
| Group B | 0 | 5 | 4 | 6 | 2.1 |

| Hand sensation measurement (after application for 6 weeks) | | | | | |
|---|---|---|---|---|---|
| | Normal | Deterioration I | Deterioration II | Deterioration III | Average value |
| Group A | 1 | 3 | 7 | 4 | 1.9 |
| Group B | 3 | 6 | 3 | 3 | 1.4 |

| Foot bottom sensation measurement (before application) | | | | | |
|---|---|---|---|---|---|
| | Normal | Deterioration I | Deterioration II | Deterioration III | Average value |
| Group A | 0 | 5 | 5 | 5 | 2.0 |
| Group B | 0 | 5 | 6 | 4 | 1.9 |

| Foot bottom sensation measurement (after application for 6 weeks) | | | | | |
|---|---|---|---|---|---|
| | Normal | Deterioration I | Deterioration II | Deterioration III | Average value |
| GroupA | 1 | 3 | 5 | 6 | 2.1 |
| Group B | 2 | 6 | 6 | 1 | 1.4 |

**TABLE 9**

| Hand sensation | | | |
|---|---|---|---|
| | Worsened | Same | Improved |
| Group A | 1 | 13 | 1 |
| Group B | 0 | 6 | 9 |

| Foot bottom sensation | | | |
|---|---|---|---|
| | Worsened | Same | Improved |
| Group A | 2 | 14 | 1 |
| Group B | 1 | 9 | 5 |

As shown in Tables 8 and 9, the sensation in the palm and the bottom of the foot tended to be improved by applying the sensation-improving cosmetic (cream) obtained in Example 5. It was thus confirmed that deterioration in sensation, particularly peripheral sensation, is expected to be improved by applying cream that includes the skin sensitivity improving agent according to the present invention.

### Test Example 7

### (Sensation-improving effect test through skin application - sphingomyelin)

Healthy elderly persons (average age: 75±3) who suffered deterioration in sensation in the hands were divided into groups A and B (one group: 15 subjects). The subjects of the group A were applied a cosmetic (cream) that was produced in the same manner as in Example 6 but did not contain a skin sensitivity improving agent once daily to whole of the hands and the feet thereof, and the subjects of the group B were applied the sensation-improving cosmetic (cream) obtained in Example 6 once daily to whole of the hands and the feet thereof. The application period was eight weeks.
The pain sensation in the palm and the arch of the foot was measured with 4 criteria (normal, deteriorations I, II, and III) before the application and after the application for 8 weeks with reference to the pain sensation in the medial side of the arm using a pain/touch-pressure sensation measuring device ("Algesiometer" manufactured by Intercross Ltd.) in accordance with the instruction manual. After the application for 8 weeks, a questionnaire survey on improvement in sensation in the hands was carried out to each elderly person. The results are shown in Tables 10 and 11. The measurement method was carried out in the same manner as that of Test Example 4.

**TABLE 10**

| Hand sensation measurement (before application) | | | | | |
|---|---|---|---|---|---|
| | Normal | Deterioration I | Deterioration II | Deterioration III | Average value |
| Group A | 0 | 5 | 5 | 5 | 2.0 |
| Group B | 0 | 5 | 5 | 5 | 2.0 |

| Hand sensation measurement (after application for 8 weeks) | | | | | |
|---|---|---|---|---|---|
| | Normal | Deterioration I | Deterioration II | Deterioration III | Average value |
| Group A | 0 | 4 | 7 | 4 | 2.0 |
| Group B | 2 | 6 | 6 | 1 | 1.4 |

| Foot bottom sensation measurement (before application) | | | | | |
|---|---|---|---|---|---|
| | Normal | Deterioration I | Deterioration II | Deterioration III | Average value |
| Group A | 0 | 6 | 4 | 5 | 1.9 |
| Group B | 0 | 4 | 5 | 6 | 2.1 |

| Foot bottom sensation measurement (after application for 8 weeks) | | | | | |
|---|---|---|---|---|---|
| | Normal | Deterioration I | Deterioration II | Deterioration III | Average value |
| Group A | 0 | 4 | 6 | 5 | 2.1 |
| Group B | 2 | 6 | 4 | 3 | 1.5 |

**TABLE 11**

| Hand sensation | | | |
|---|---|---|---|
| | Worsened | Same | Improved |
| Group A | 1 | 13 | 1 |
| Group B | 0 | 5 | 10 |

| Foot bottom sensation | | | |
|---|---|---|---|
| | Worsened | Same | Improved |
| Group A | 2 | 11 | 2 |
| Group B | 1 | 8 | 6 |

As shown in Tables 10 and 11, the sensation in the palm and the bottom of the foot tended to be improved by applying the sensation-improving cosmetic (cream) obtained in Example 6. It was thus confirmed that deterioration in sensation, particularly peripheral sensation, is expected to be improved by applying cream that includes the skin sensitivity improving agent according to the present invention.

### Example 7

### (Preparation of sensation-improving liquid nutrient composition - milk-derived phospholipid-containing composition)

50 g of the milk-derived phospholipid-containing composition obtained in Example 1 was dissolved in 4950 g of deionized water. The solution was heated to 50°C, and stirred at 6000 rpm for 30 minutes using a TK-homomixer ("TK ROBO MICS" manufactured by PRIMIX Corporation) to obtain a milk-derived phospholipid-containing composition solution having a milk-derived phospholipid content of 310 mg/100 g. 5.0 kg of casein, 5.0 kg of a soybean protein, 1.0 kg offish oil, 3.0 kg of perilla oil, 18.0 kg of dextrin, 6.0 kg of a mineral mixture, 1.95 kg of a vitamin mixture, 2.0 kg of an emulsifying agent, 4.0 kg of a stabilizer, and 0.05 kg of essence were added to 4.0 kg of the milk-derived phospholipid-containing composition solution. A retort pouch (200 ml) was charged with the mixture. The pouch was then sterilized at 121°C for 20 minutes using a retort sterilizer (class-1 pressure vessel, "RCS-4CRTGN" manufactured by Hisaka Works, Ltd.) to produce 50 kg of a sensation-improving liquid nutrient composition according to the present invention. The sensation-improving liquid nutrient composition contained the milk-derived phospholipid in an amount of 24.8 mg/100 g.

### Example 8

### (Preparation of sensation-improving liquid nutrient composition - sphingomyelin)

50 g of a sphingomyelin material ("Phospholipid 700" manufactured by Fonterra, sphingomyelin content: 25%) was dissolved in 4950 g of deionized water. The solution was heated to 50°C, and stirred at 6000 rpm for 30 minutes using a TK-homomixer ("TK ROBO MICS" manufactured by PRIMIX Corporation) to obtain a sphingomyelin solution (skin sensitivity improving agent) with a sphingomyelin content of 250 mg/100 g. 5.0 kg of casein, 5.0 kg of a soybean protein, 1.0 kg offish oil, 3.0 kg of perilla oil, 18.0 kg of dextrin, 6.0 kg of a mineral mixture, 1.95 kg of a vitamin mixture, 2.0 kg of an emulsifying agent, 4.0 kg of a stabilizer, and 0.05 kg of essence were added to 4.0 kg of the sphingomyelin solution. A retort pouch (200 ml) was charged with the mixture. The pouch was then sterilized at 121 °C for 20 minutes using a retort sterilizer (class-1 pressure vessel, "RCS-4CRTGN" manufactured by Hisaka Works, Ltd.) to produce 50 kg of a sensation-improving liquid nutrient composition according to the present invention. The sensation-improving liquid nutrient composition contained sphingomyelin in an amount of 20 mg/100 g.

### Example 9

### (Preparation of sensation-improving gel food - milk-derived phospholipid-containing composition)

10 g of the milk-derived phospholipid-containing composition obtained in Example 1 was dissolved in 700 g of deionized water. The solution was heated to 50°C, and stirred at 9500 rpm for 30 minutes using an ultra-disperser ("ULTRA-TURRAX T-25" manufactured by IKA Japan). 40 g sorbitol, 2 g of a sour agent, 2 g of essence, 5 g of pectin, 5 g of a milk serum protein concentrate, 1 g of calcium lactate, and 235 g of deionized water were added to the solution. After stirring the mixture, a cheer pack (200 ml) was filled with the mixture. After sterilization at 85°C for 20 minutes, the pack was sealed to obtain five bags (200 g) of sensation-improving gel food according to the present invention. The sensation-improving gel food thus obtained did not undergo precipitation, and did not have an abnormal flavor. The sensation-improving gel food contained the milk-derived phospholipid in an amount of 310 mg/100 g.

### Example 10

### (Preparation of sensation-improving gel food - sphingomyelin)

10 g of a sphingomyelin material ("Phospholipid 500" manufactured by Fonterra, sphingomyelin content: 10%) was dissolved in 700 g of deionized water. The solution was heated to 50°C, and stirred at 9500 rpm for 30 minutes using an ultra-disperser ("ULTRA-TURRAX T-25" manufactured by IKA Japan) to obtain a skin sensitivity improving agent containing sphingomyelin as an active ingredient.

40 g sorbitol, 2 g of a sour agent, 2 g of essence, 5 g of pectin, 5 g of a milk serum protein concentrate, 1 g of calcium lactate, and 235 g of deionized water were added to the skin sensitivity improving agent. After stirring the mixture, a cheer pack (200 ml) was filled with the mixture. After sterilization at 85°C for 20 minutes, the pack was sealed to obtain five bags (200 g) of sensation-improving gel food according to the present invention. The resulting sensation-improving gel food did not undergo precipitation, and did not have an abnormal flavor. The sensation-improving gel food contained sphingomyelin in an amount of 100 mg/100 g.

### Example 11

### (Preparation of sensation-improving drink - milk-derived phospholipid-containing composition)

2 g of a sour agent was dissolved in 700 g of deionized water. 10 g of the milk-derived phospholipid-containing composition obtained in Example 2 was then dissolved in the solution. The resulting solution was heated to 50°C, and stirred at 9500 rpm for 30 minutes using an ultra-disperser ("ULTRA-TURRAX T-25" manufactured by IKA Japan). After the addition of 100 g of maltitol, 20 g of reduced starch syrup, 2 g of essence, and 166 g of deionized water, a glass bottle (100 ml) was filled with the mixture. After sterilization at 90°C for 15 minutes, the bottle was sealed to obtain ten bottles (100 ml) of sensation-improving drink. The resulting sensation-improving drink did not undergo precipitation, and did not have an abnormal flavor. The sensation-improving drink contained the milk-derived phospholipid in an amount of 380 mg/100 g.

### Example 12

### (Preparation of sensation-improving drink - sphingomyelin)

2 g of a sour agent was dissolved in 700 g of deionized water. 10 g of a sphingomyelin material ("Phospholipid 700" manufactured by Fonterra, sphingomyelin content: 25%) was then dissolved in the solution. The resulting solution was heated to 50°C, and stirred at 9500 rpm for 30 minutes using an ultra-disperser ("ULTRA-TURRAX T-25" manufactured by IKA Japan) to obtain a skin sensitivity improving agent containing sphingomyelin as an active ingredient. After the addition of 100 g of maltitol, 20 g of reduced starch syrup, 2 g of essence, and 166 g of deionized water to the skin sensitivity improving agent, a glass bottle (100 ml) was filled with the resulting mixture. The bottle was sterilized at 90°C for 15 minutes, sealed, whereby preparing ten bottles (100 ml) of sensation-improving drink. The resulting sensation-improving drink did not undergo precipitation, and did not have an abnormal flavor. The sensation-improving drink contained sphingomyelin in an amount of 250 mg/100 g.

### Example 13

### (Preparation of skin sensitivity improving agent (tablet) - milk-derived phospholipid-containing composition)

The raw materials were mixed in a ratio shown in Table 12. 1 g of the resulting mixture was formed and tableted by a conventional method to produce a skin sensitivity improving agent according to the present invention. The skin sensitivity improving agent contained the milk-derived phospholipid in an amount of 31 mg/l g.

**TABLE 12**

| Tablet composition (wt%) | |
|---|---|
| Hydrated crystalline glucose | 83.5 |
| Milk-derived phospholipid-containing composition (Example 1) | 10.0 |
| Mineral mixture | 5.0 |
| Sugar ester | 1.0 |
| Essence | 0.5 |

### Example 14

### (Preparation of skin sensitivity improving agent (tablet) - sphingomyelin)

The raw materials were mixed in a ratio shown in Table 13. 1 g of the resulting mixture was formed and tableted by a conventional method to produce a skin sensitivity improving agent (tablet) according to the present invention. The skin sensitivity improving agent contained sphingomyelin in an amount of 10 mg/l g.

**TABLE 13**

| Tablet composition (wt%) | |
|---|---|
| Hydrated crystalline glucose | 83.5 |
| Sphingomyelin material (content: 10%, | 10.0 |
| Phospholipid 500, Fonterra) Mineral mixture | 5.0 |
| Sugar ester | 1.0 |
| Essence | 0.5 |

### Example 15 5

### (Preparation of sensation-improving cosmetic (lotion) - milk-derived phospholipid-containing composition)

A sensation-improving cosmetic (lotion) was produced by mixing the raw materials in a ratio shown in Table 14.

**TABLE 14**

| | |
|---|---|
| Sorbitol | 3.0 |
| Sodium DL-pyrrolidone carboxylate | 2.0 |
| Carboxymethyl cellulose | 0.3 |
| Parabene | 0.1 |
| Milk-derived phospholipid-containing composition (Example 2) | 1.5 |
| Essence | Proper quantity |
| Sterilized ion-exchanged water | Balance (total: 100) |

### Example 16

### (Preparation of sensation-improving cosmetic (lotion) - sphingomyelin)

A sensation-improving cosmetic (lotion) was produced by mixing the raw materials in a ratio shown in Table 15.

**TABLE 15**

| | |
|---|---|
| Sorbitol | 3.0 |
| Sodium DL-pyrrolidone carboxylate | 2.0 |
| Carboxymethyl cellulose | 0.3 |
| Parabene | 0.1 |
| Sphingomyelin (Example 3) | 0.1 |
| Essence | Proper quantity |
| Sterilized ion-exchanged water | Balance (total: 100) |

### Example 17

### (Preparation of sensation-improving feed - milk-derived phospholipid-containing composition)

2 kg of the milk-derived phospholipid-containing composition obtained in Example 2 was dissolved in 98 kg of deionized water. The resulting solution was heated to 50°C, and stirred at 3600 rpm for 40 minutes using a TK-homomixer ("MARK II 160" manufactured by PRIMIX Corporation) to obtain a milk-derived phospholipid solution (milk-derived phospholipid content: 760 mg/100 g). 12 kg of soybean cake, 14 kg of skim milk powder, 4 kg of soybean oil, 2 kg of corn oil, 23.2 kg of palm oil, 14 kg of corn starch, 9 kg of flour, 2 kg of bran, 5 kg of a vitamin mixture, 2.8 kg of cellulose, and 2 kg of a mineral mixture were added to 10 kg of the milk-derived phospholipid solution. The mixture was sterilized at 120°C for 4 minutes to prepare 100 kg of a sensation-improving dog food according to the present invention. The sensation-improving dog food contained the milk-derived phospholipid in an amount of 76 mg/100 g.

### Example 18

### (Preparation of sensation-improving feed - sphingomyelin)

2 kg of a sphingomyelin material ("SM-4" manufactured by Corman, sphingomyelin content: 4%) was dissolved in 98 kg of deionized water. The solution was heated to 50°C, and stirred at 3600 rpm for 40 minutes using a TK-homomixer ("MARK II 160" manufactured by PRIMIX Corporation) to obtain a skin sensitivity improving agent (sphingomyelin content: 80 mg/100 g). 12 kg of soybean cake, 14 kg of skim milk powder, 4 kg of soybean oil, 2 kg of corn oil, 23.2 kg of palm oil, 14 kg of corn starch, 9 kg of flour, 2 kg of bran, 5 kg of a vitamin mixture, 2.8 kg of cellulose, and 2 kg of a mineral mixture were added to 10 kg of the skin sensitivity improving agent. The mixture was sterilized at 120°C for 4 minutes to prepare 100 kg of a sensation-improving dog food according to the present invention. The sensation-improving dog food contained sphingomyelin in an amount of 8 mg/100 g.

## Claims

1. A skin sensitivity improving agent comprising a phospholipid as an active ingredient.

2. A skin sensitivity improving agent comprising a milk-derived phospholipid as an active ingredient.

3. The skin sensitivity improving agent according to claim 2, wherein the milk-derived phospholipid is a composition (milk-derived phospholipid-containing composition) that is obtained from milk or a milk material and contains a phospholipid in an amount of 10 wt% or more based on the total solid content.

4. The skin sensitivity improving agent according to claim 2, wherein the milk-derived phospholipid-containing composition is obtained by treating milk or a milk material using a microfiltration (MF) membrane having a pore diameter of 0.1 to 2.0 µm or an ultrafiltration (UF) membrane having a molecular weight cut-off of 5 to 500 kDa.

5. The skin sensitivity improving agent according to claim 2, wherein the milk-derived phospholipid-containing composition is obtained by adding an acid to milk or a milk material to adjust the pH to 4.0 to 5.0, removing a casein protein as a precipitate, and treating the resultant using an MF membrane having a pore diameter of 0.1 to 2.0 µm or a UF membrane having a molecular weight cut-off of 5 to 500 kDa.

6. The skin sensitivity improving agent according to any one of claims 3 to 5, wherein the milk or the milk material is butter serum or buttermilk.

7. A skin sensitivity improving agent comprising a sphingosine-containing phospholipid and/or a derivative thereof as an active ingredient.

8. The skin sensitivity improving agent according to claim 7, wherein the sphingosine-containing phospholipid is sphingomyelin.

9. A sensation-improving food, drink, feed, or cosmetic comprising the skin sensitivity improving agent according to any one of claims 1 to 8.

10. A method of producing a skin sensitivity improving agent that comprises a milk-derived phospholipid-containing composition as an active ingredient, the method comprising treating milk or a milk material using a microfiltration (MF) membrane having a pore diameter of 0.1 to 2.0 µm or an ultrafiltration (UF) membrane having a molecular weight cut-off of 5 to 500 kDa to obtain the milk-derived phospholipid-containing composition.

11. The method according to claim 10, further comprising adding an acid to the milk or the milk material to adjust the pH to 4.0 to 5.0, and removing a casein protein as a precipitate before treating the milk or the milk material using the MF membrane or the UF membrane.
